# EUROPEAN PATENT SPECIFICATION

(11) **EP 1 418 853 B1**
(45) Date of publication and mention of the grant of the patent: **16.02.2011**
(21) Application number: 01966681.7
(22) Date of filing: 13.08.2001
(51) Int. Cl.: A61B 17/70

(54) **VERTEBRAL ALIGNMENT AND FIXATION ASSEMBLY**
WIRBELAUSRICHTUNGS- UND FIXIERUNGSANORDNUNG
ALIGNEMENT VERTEBRAL ET ENSEMBLE DE FIXATION

(43) Date of publication of application: 19.05.2004
(73) Proprietor: McKinley, Laurence M., Rancho Santa FE, CA 92067 (US)
(72) Inventor: McKinley, Laurence M., Rancho Santa FE, CA 92067 (US)
(74) Representative: Newstead, Michael John
(86) International application number: PCT/US2001/041684
(87) International publication number: WO 2003/015648

(56) References cited:
- WO-A-01/08574
- WO-A-98/27884
- US-A- 5 443 467
- US-A- 5 882 350
- US-A- 5 882 350
- US-A- 6 010 503
- US-A- 6 132 432
- US-A- 6 132 434
- US-B1- 6 280 442

## Description

### FIELD OF THE INVENTION

The present invention relates generally to an apparatus for alignment and fixation of vertebral bodies.

### BACKGROUND OF THE INTENTION

Pedicle screws allow spine surgeons to attach rods or plates to the thoracic and lumbar spine. This rigidly immobilizes the spine segments, promoting the bone graft to grow into a fusion, welding spinal segments into one solid unit, reducing pain and stabilizing deformity without requiring complete immobilization of the patient for the extended period of time during the healing process.

While many different pedicle screws have been developed, presently most pedicle screws are fixed axis devices which must be carefully aligned during insertion and fixation in the spine. Specifically, the screws must be drilled or screwed into the bone at a very specific angle to assure that the alignment hardware is exactly positioned such that the receiving portions of the fixation hardware are aligned so that the rod can be passed therethrough without distorting the screw or putting an undesirable level of stress on the attachment point. As a result, the alignment procedure requires a considerable amount of time, increasing the possibilities of complications during surgery and, in many cases the alignment fails and must be repeated. Further, the insertion of the screw is dependent on the angle of alignment required, resulting in insertions that are not in the most secure or safe positions with respect to the vertebral bodies.

The art contains a variety of pedicle screws which permit a level of freedom with respect to the alignment of the screw and the coupling element. However, these teachings have generally been complex, and inadequately reliable with respect to durability. The considerable drawbacks associated with the prior art systems include limited angular adjustability, complexity, difficult of properly positioning the coupling elements and the rod, tedious manipulation of the many parts associated with the complex devices and the considerable cost associated with manufacturing such complex mechanisms.

WO 01/08574 discloses a bone screw which comprises a thread section and a receiving part for receiving a bar which is to be connected to the bone screw. An external nut connects the bone screw and bar and secures said connection. In order for the connection to have sufficient rigidity and durability, the interior of the external nut has a sleeve-shaped element with a predetermined internal dimension, whose external diameter is almost equal to, or slightly less than the diameter of the bore. The sleeve-shaped element contains a pressure element. Said pressure element has a first section on its end facing the base of the bore, whose external dimensions are greater than the predetermined internal dimensions and which causes the element to expand by the exertion of pressure on the bar to be received.

WO 98/27884 discloses a pedicle screw assembly for use with a rod for the immobilization of bone segments. The assembly is composed of a screw, a poly-axial housing, a washer, a set screw, and a cup shaped washer. When the screw is placed inside the poly-axial housing, the head of the screw comes into contact with a middle section of the poly-axial housing, and is secured into the bone so that the poly-axial housing is pivotable. The housing includes a pair of upstanding posts with interior threads. A washer is inserted between the head of the screw and the rod. A cap, having a bottom with a pair of openings and a lateral cross connector, is placed over the posts so that the cross connector engages the rod. The cross connector and washer have semi-cylindrical rod engaging surfaces. A set screw is threaded into the housing posts to secure the rod.

Accordingly, a need exists for an inexpensive, durable and simple vertebral alignment assembly that allows a surgeon to freely manipulate the alignment of the coupling hardware such that the fixation rods can be properly positioned with respect to the vertebral bodies without a time consuming and potentially dangerous alignment procedure.

### SUMMARY OF THE INVENTION

The present invention is defined in claim 1 and relates generally to an apparatus for aligning and fixing vertebral bodies. More specifically, the present invention is directed to a vertebral alignment/fixation assembly which allows a surgeon to manipulate and align the unit coupling the fixation hardware with the pedicle screw, the assembly comprising a hemispherical headed pedicle screw disposed within a slotted coupling unit designed to allow angular adjustment of the pedicle screw up to 90° and which may be securely locked into position via a single threaded locking nut once a standard alignment rod has been inserted into the slotted coupling unit. The vertebral alignment/fixation assembly enabling the angular adjustment of the fixation system hardware after final placement and insertion of the pedicle screw into the vertebral body.

In one embodiment, the vertebral alignment/fixation assembly of the current invention generally consists of three main components: a hemispherical pedicle screw, a slotted coupling unit designed to receive the pedicle screw and an alignment rod, and a securing nut for fixing the angular position of the coupling unit and the position of the alignment rod within the coupling unit.

In one alternative exemplary embodiment, the pedicle screw of the invention has a slotted tip to allow the screw to self-tap the vertebral body and thereby ease the insertion of the screw into the bone.

In another exemplary embodiment the portion of the securing nut which engages the alignment rod is textured to provide a more secure grip of the alignment rod.

In still another exemplary embodiment the securing nut has an annular channel disposed such that a screw driver can be inserted therethrough and interact with the pedicle screw to drive the screw into a vertebral body.

In yet another exemplary embodiment the pedicle screw is provided with a square opening in its hemispherical head such that a square headed driving tool can be mated therewith to drive the screw into the vertebral body.

In still yet another exemplary embodiment the components of the system are made from an orthopaedically suitable material, such as, for example, stainless steel or titanium.

In still yet another preferred embodiment, the invention is directed to a system for aligning and fixing vertebral bodies comprising a multiplicity of vertebral alignment components as described above attached at suitable points of attachment as determined by the deformity of the spine.

The invention may be used in a method for aligning vertebral bodies. The method comprises manipulating, aligning and fixing the spine using a vertebral alignment system as described above.

### BRIEF DESCRIPTION OF THE DRAWINGS

These and other features and advantages of the present invention will be better understood by reference to the following detailed description when considered in conjunction with the accompanying drawings wherein:
FIG. 1 is a side view of an embodiment of an unassembled pedicle screw according to the invention.
FIG. 2a is a side view of an embodiment of a partially assembled pedicle screw according to the invention.
FIG. 2b is a front view of an embodiment of a partially assembled pedicle screw according to the invention.
FIG. 2c is a front partial cross-sectional view of an embodiment of a partially assembled pedicle screw according to the invention.
FIG. 3a is a top view of an embodiment of a securing nut according to the invention.
FIG. 3b is a cross section of an embodiment of a securing nut according to the invention.
FIG. 3c is a side view of an embodiment of a securing nut according to the invention.
FIG. 4a is a side view of the interrelation of an embodiment of a pedicle screw and screw driver according to the invention.
FIG. 4b is a cross section of the interrelation of an embodiment of a pedicle screw and screw driver according to the invention.
FIG. 5 is a side view of an assembled pedicle screw according to the invention.
FIG. 6 is a schematic view of the manipulation and alignment of the spine utilizing an embodiment of the vertebral alignment/fixation system according to the invention.
FIG. 7 is a schematic view of the manipulation and alignment of the spine utilizing an embodiment of the vertebral alignment/fixation system according to the invention.

### DETAILED DESCRIPTION OF THE INVENTION

The present invention relates generally to an apparatus for aligning and fixing vertebral bodies. More specifically, the present invention is directed to a system which allows a surgeon to manipulate the angular alignment of the fixation hardware of a vertebral alignment/fixation system after insertion and fixation of the pedicle screws into the vertebral bodies using freely rotatable couplers mounted to hemispherical pedicle screws. The system is further designed to enable the fixation of the angular alignment and installation and fixation of the alignment rods to the couplers by application of a single securing nut.

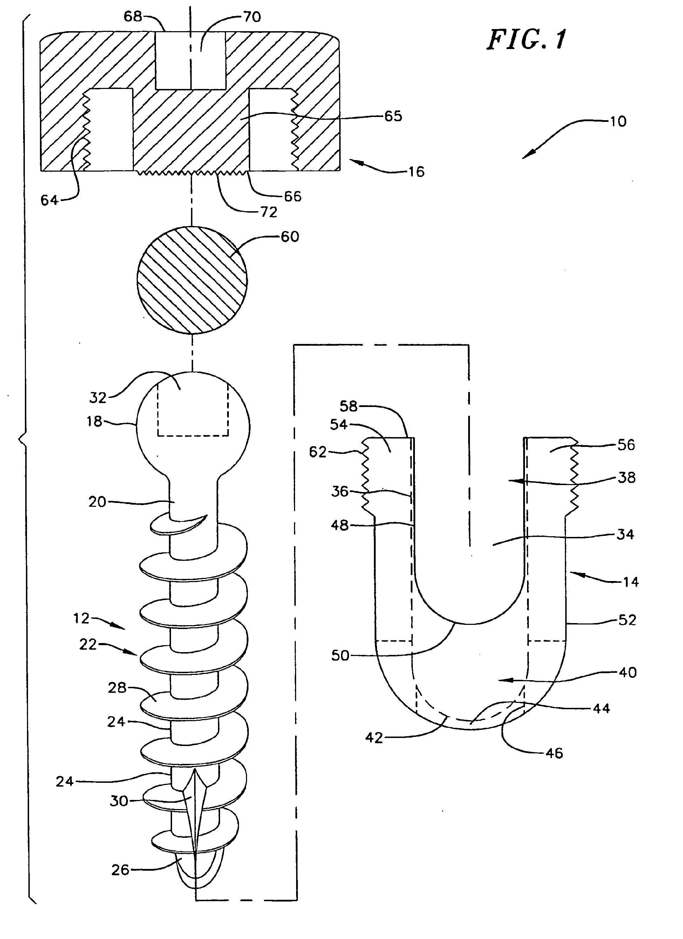

As shown in FIGs 1 to 5, the vertebral alignment/fixation assembly **10** of the current invention consists of three main components: a hemispherical pedicle screw **12,** a coupler unit **14** which functions as a universal joint, and a securing nut **16.**

The hemispherical pedicle screw **12,** as shown in FIG. 1, comprises a substantially hemispherical head portion **18,** a neck portion **20** and a shaft portion **22.** Although in FIG. 1 the shaft **22** is shown as having a generally cylindrical body **24** and a tapered tip **26** with a thread **28** dispose along the length of the shaft **22,** any shaft design, thread pitch or tip taper suitable for insertion into a vertebral body can be utilized in the current invention. In the embodiment shown in FIG. 1, the tapered tip **26** of the pedicle screw shaft **22** further comprises a slotted groove **30** running longitudinally along the shaft, designed such that the screw is self tapping easing the insertion of the pedicle screw **12** into the vertebral body.

The head portion **18** of the pedicle screw **12** comprises a substantially hemispherical shape. The substantially hemispherical shape of the head portion **18** of the screw **12** is a portion or section of a sphere. Although in the embodiment shown, the section or portion of the sphere comprising the head **18** of the screw **12** is greater in extent than a hemisphere, it should be understood that any external contour which is equidistant from a center point of the head portion **18** could be utilized. In the embodiment shown in FIG. 1, the major cross-section of the substantially hemispherical head portion **18** includes at least 270 degrees of a circle.

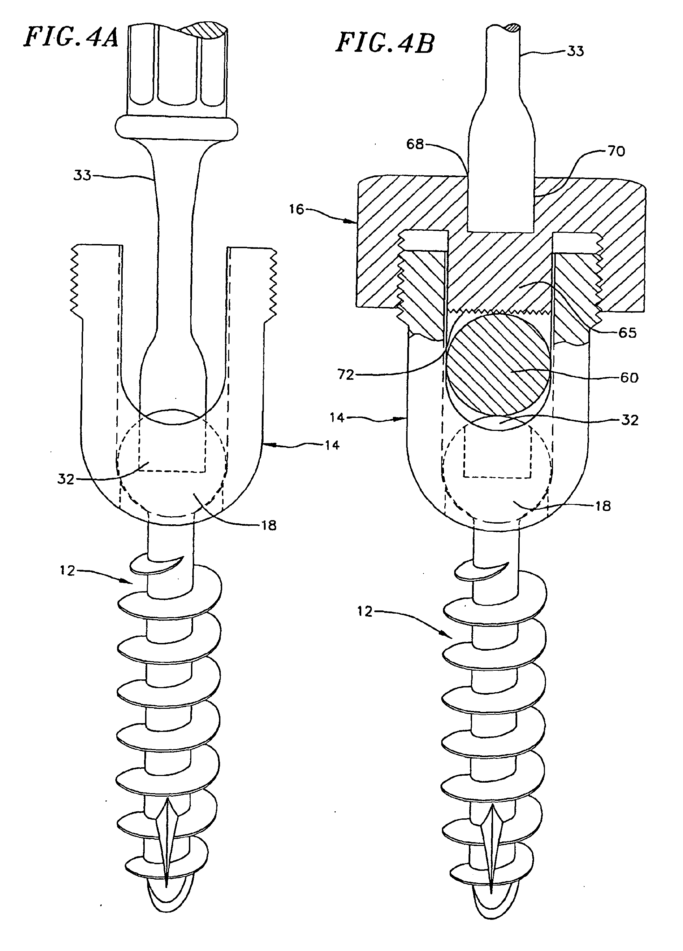

The hemispherical head portion **18** also has a recess **32** disposed therein (shown in phantom in FIG. 1). The recess **32** defines a engagement point for the application of torque from a torque driving tool **33** for driving the screw **12** into a bone. The specific shape of the recess **32** may be chosen to cooperate with any suitable screw-driving tool **33,** as shown in FIGs. 4a and 4b. For example, the recess **32** may comprise a slot for a flat-headed screwdriver, a crossed recess for a phillips head screwdriver, a hexagonally shaped hole for receiving an allen wrench, or a "figure-8" shaped driver. In a preferred embodiment, a square-headed hole for a square screwdriver or socket-type wrench is utilized. Although the recess **32** is shown to be co-axial with the general elongate axis of the screw shaft **22,** it should be understood that any arrangement of recess **32** and screw **12** can be utilized such that sufficient torque may be applied to the screw **12** to drive it into a bone.

The head portion **18** of the screw **12** is connected to the shaft portion **22** at a neck portion **20.** In relation to each other, the diameter of the shaft **22** should be less than the diameter of the semi-spherical head **18,** and the neck **20** of the screw **12** should be preferably narrower than the widest portion of the shaft **22.** A pedicle screw **12** according to the invention having such dimensional relationships is preferable because the screw may be locked at a variety of angles with relation to the coupling unit **14** while still being securely joined to the coupling element **14** (embodiments of which are shown in FIGs. 1, 2 and 5). Although any biocompatible material having suitable strength and durability characteristics may be utilized, the pedicle screw **12** is preferably made from surgical grade titanium or stainless steel.

One exemplary embodiment of the universal joint coupling element **14** of the present invention is shown in a side view in FIG. 1, critical elements are shown in phantom. The coupling element **14** comprises a generally cylindrical tubular body which defines an inner passage **34** having an inner wall **36.** The inner passage **34** comprises an upper generally cylindrical portion **38** and an inwardly curved lower portion **40.** The inwardly curved lower portion **40** defines a socket, into which the head **18** of the screw **12** may rotatingly engage. The bottom surface **42** of the coupling element **14** includes an opening **44** defining a passage **46** such that the shaft **22** of the screw **12** may extend therethrough and pass outside the body of the coupling element **14.** To securely engage the screw **12** within the coupling element **14,** the dimensions of the opening **44** and passage **46,** must be greater than the diameter of the shaft **22** of the screw **12,** but less than the largest diameter of the head **18.**

The cylindrical upper portion **38** of the coupling element **14** includes a pair of vertically oriented, channels **48** having rounded bottom surfaces **50** and open top portions disposed on opposing sides of the coupling element **14.** In combination the channels **48** form engagement point for an elongated fixation rod **60.** In addition the channels **48** divide the wall **52** of the coupling element **14** into upwardly extending members **54** and **56**. As shown in the embodiment illustrated in FIGs. 1, 2 and 5, the vertical distance from the top **58** of the channels **48** to the curved bottom **50,** is sufficient to allow the rod **60** which is to be provided to slidingly engage therein such that the rod **60** may be fully nested in the channels **48.** In addition, the curved bottom **50** of the channels **48** are arranged such that the top of the head **18** of the screw **12,** when fully nested in the lower socket portion **40,** extends above the edge of the curved bottom **50** of the channels **48** such that a rod **60** positioned therein will pressingly engage the head portion **18** of the screw **12.** The top **58** of the upper portion **38** of the coupling element **14,** which comprises upwardly extending members **54** and **56,** have disposed thereon a threading **62.** The upper portion **38,** and the threading **62** thereon, is ideally suited for threadingly engage a securing nut **16.**

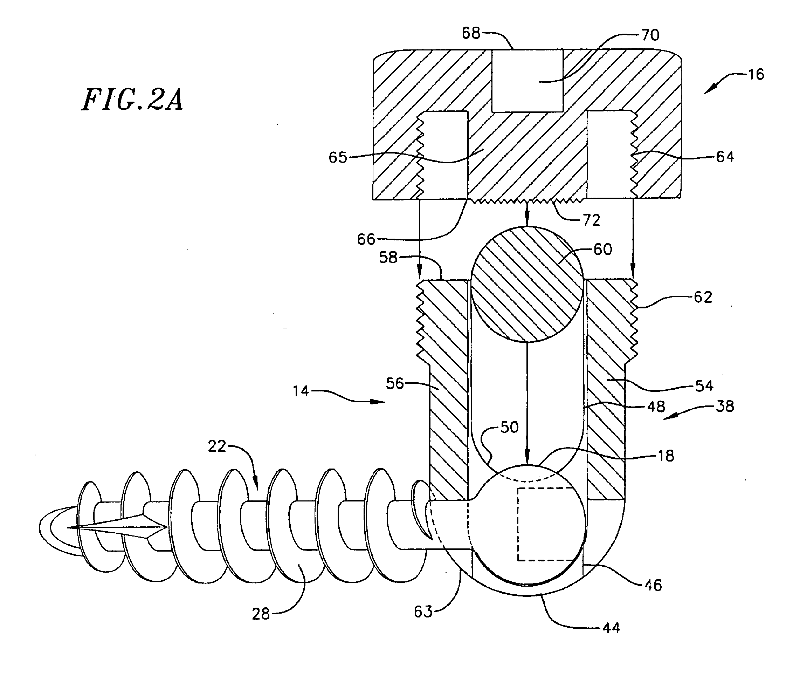

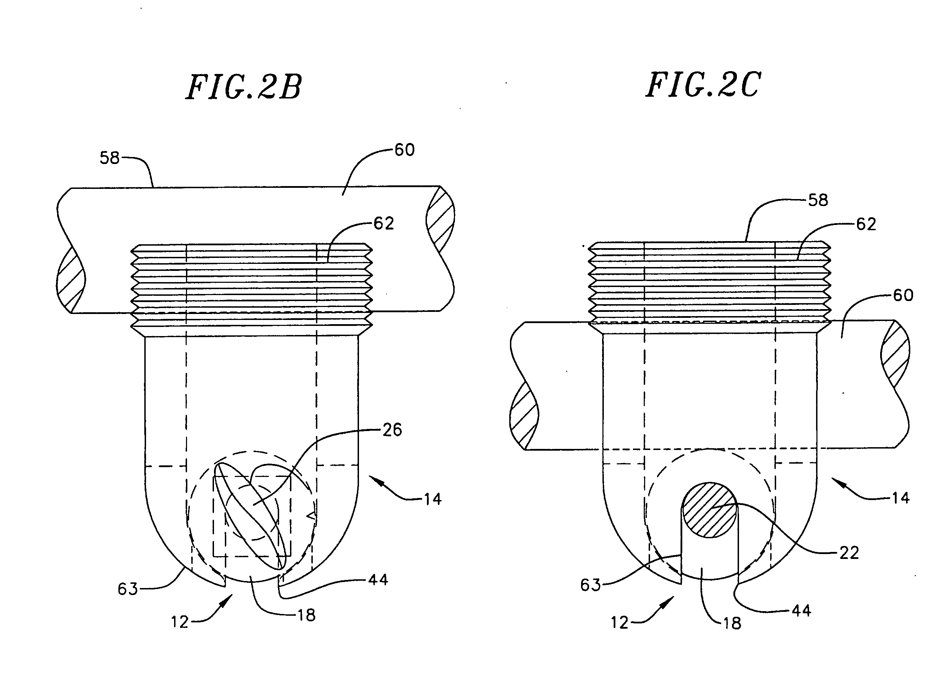

FIGs. 2a, 2b and 2c show an additional feature of one exemplary embodiment of the invention, which allows angular alignment of the screw **12** up to at least a 90 degree angle with respect to the coupling element **14.** In this embodiment the lower portion **40** of the coupling unit **14** further comprises a pair of lower slots **63** extending from the opening **44** and passage **46** in the bottom surface **42** of the coupling unit **14.** The lower slots **63** are aligned on opposite sides of the bottom surface **42** of the coupling unit **14** such that in combination the slots **63** define a single 180 degree passage dimensioned to allow the neck portion **20** of the screw **12** to move therein when the head portion **18** of the screw is fully engaged in the socket **40** of the coupling unit **14** and the coupling unit **14** is properly oriented with respect to the shaft **22** of the screw **12.** As shown, even in this extreme angular position, the coupling unit **14** is designed such that a rod **60** inserted into the channels **48** will press against the head portion **18** of the screw **12** and at the urging of the securing nut **16** engage and fix both the rod 60 and in turn the coupling unit **14** into alignment.

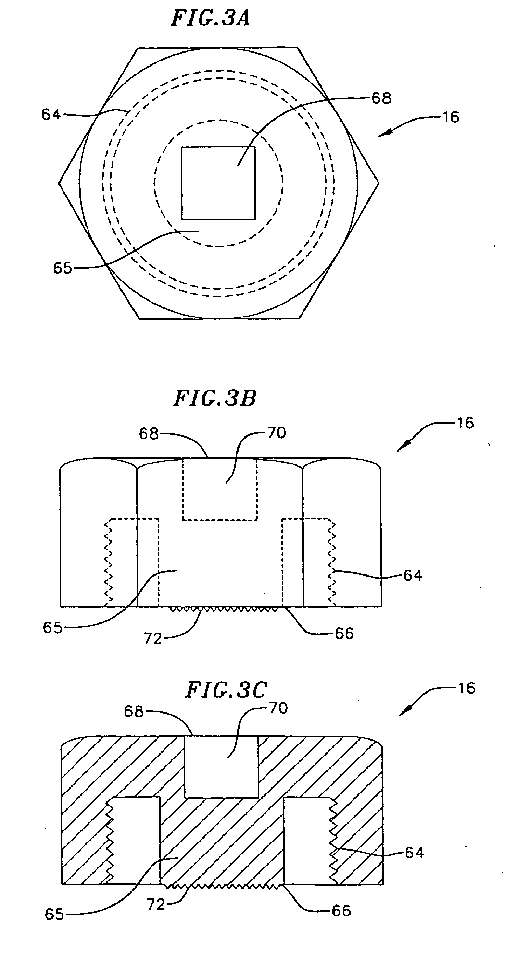

The top securing nut **16** is shown in top view in FIG. 3a, in side view in FIG. 3b, and in cross section in FIG. 3c. The nut **16** comprises an inner threading **64,** which is intended to mate with the threading **62** on the upwardly extending members **54** and **56** of the upper portion **38** of the coupling element **14.** The nut **16** also comprises an inner plug portion **65** having a bottom surface **66** which is intended to seat against the top surface of the rod **60** seated in the coupling element **14,** providing a means for driving the rod **60** downward and against the head portion **18** of the screw **12.** A central annular opening **68** is provided in the center of the nut **16** defining a passage **70** passing therethrough, the passage **70** and opening **68** being designed such that the screw driver utilized to drive the screw **12** into the vertebral body can fit therein and can be utilized to tighten the nut **16** onto the coupling unit **14,** as shown in FIG. 4b. Although the embodiment of the passage **70** shown in FIGs. 3a to 3c terminates in the middle of the plug **65** of the nut **16,** the passage **70** could also transect the plug **65** forming a conduit betwwen the opening **68** and the bottom surface **66.**

In the preferred embodiment shown in FIG. 1, the bottom surface **66** of the nut **16** further comprises a plurality of raised metal teeth **72** designed to bitingly engage and press into the rod **60** providing additional frictional engagement between the rod **60** and the vertebral alignment assembly **10** such that the possibility of a mechanical shock jarring the rod **60** loose from the vertebral alignment assembly **10** is reduced. In another preferred embodiment, the rod 60 is manufactured with a rolled or corrugated finish to improve the frictional engagement between the rod **60** and the teeth **72** on the bottom surface **66** of the nut **16.**

FIGs. 4a and 4b show the engagement of a driver **33** with the vertebral alignment assembly **10** to first engage the screw **12** into the vertebral body **74,** as shown in FIGs. 4a, and then to engage the securing nut **16** onto the coupling unit **14,** as shown in FIG. 4b. As shown in FIGs. 4a, the coupler unit **14** is designed such that the screw driver **33** can fit inside the inner passage **34** of the coupler **14** and engage the recess **32** of the head portion **18** of the screw **12** to drive the screw **12** into the vertebral body **74.** As shown in FIG. 4b, the securing nut **16** is further designed such that the screw driver **33** can engage nut opening **68** to secure the nut **16** on the coupling unit **14** and thereby fix alignment rod **60** into coupling unit **14** and further secure the alignment of the coupling unit **14** in relation to the axis of the screw **12.**

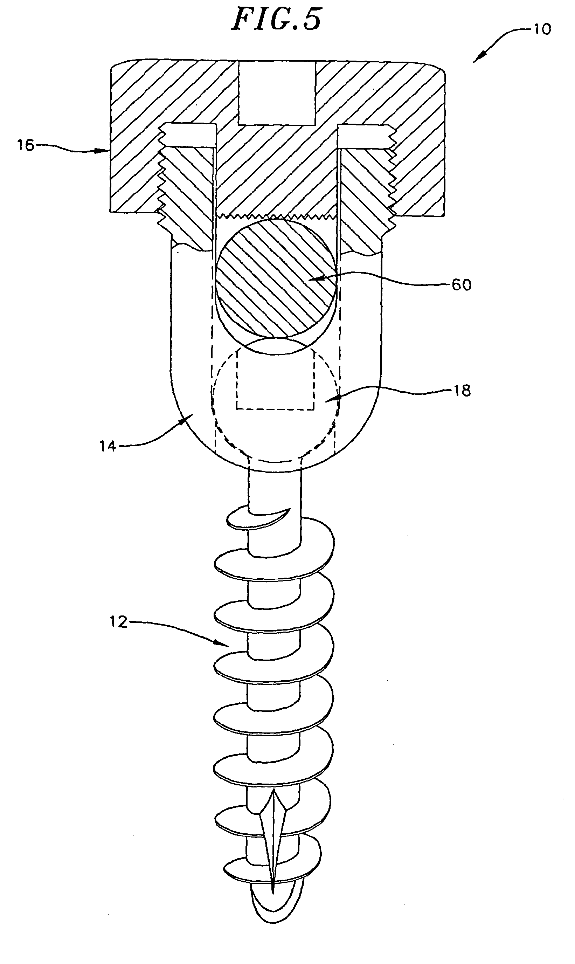

Referring now to FIG. 5, the coupling element **14** is shown with the screw **12** inserted therethrough, and the head **18** of the screw **12** nested in the lower socket portion **40** of the coupling element **14.** The shaft portion **22** of the screw **12** is inserted downward, through the interior passage **34** of the coupling element **14,** and out through the opening **44.** In this position, the curved undersurface of the head portion **18** rests against the inwardly curved bottom surface **42** of the lower socket portion **40,** and is prevented from translating further downward by the dimensions of the opening **44.** Meanwhile, the uppersurface of the head portion **18** is pressed against the rod **60** which is pressed into the head portion **18** by securing nut 16 thereby simultaneously preventing the rod **60** from moving out of the coupling unit **14** and preventing the coupling unit **14** from moving relative to the screw **12.**

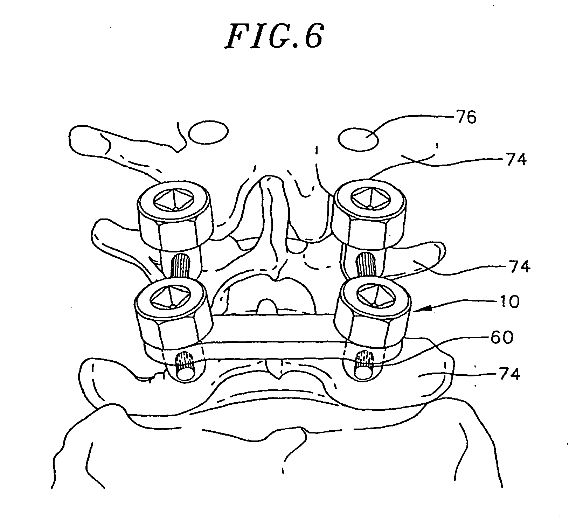

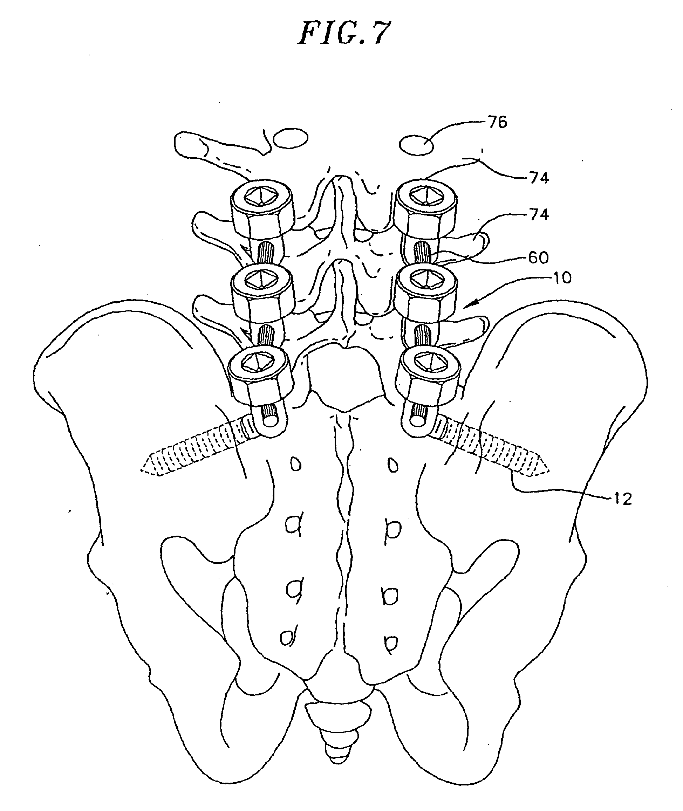

FIGs. 6 and 7, show a side view of the fully locked coupling element, rod, and screw system in relation to a vertebral body **74.** FIG. 6 shows the vertebral alignment/fixation assembly **10** of the invention anchored traditionally in a plurality of vertebral bodies with an optional crosslink stabilizing bar. FIG. 7 shows the vertebral alignment/fixation assembly **10** of the invention anchored into the ileum bone with the screw **12** aligned at a 90° angle with reference to the coupling unit **14.** With reference to these Figures, the preferred method of assembly and alignment of vertebral bodies is described.

First, a pre-drilled hole **76** is provided in the bone **74,** into which the screw **12** is to be anchored. The hole **76** may be pretapped, or, as described above, the external threading **28** at the tip portion **24** of the screw **12** may include a self-tapping slot **30.** In either case, during assembly, the tip **26** of the screw **12** is inserted through the interior passage **34** of the coupling element **14** until the shaft **22** of the screw **22** extends out of the coupling element **14** and the head 18 of the screw **12** is engaged in the lower socket portion **40** of the coupling unit **14.** At this point in the assembly process, the coupling element **14** has the capacity to rotate relative to the screw **12.** A screw-driving tool **33** is then aligned with the recess **32** in the head **18** of the screw **12** so that it may be driven into the preformed hole **76** in the bone **74.**

After the screw **12** has been driven into the hole **76,** the coupling element may be rotated relative to the screw **12,** to align the coupling element **14** such that a support rod **60** may be engaged within the rod receiving channel **48** and properly aligned according to the surgeon's wishes. As shown best in FIG. 5, and previously discussed, the bottom of the rod **60** seats on the top of the head portion **18** of the screw **12,** and not fully on the bottom curved surface **50** of the channels **48.**

After the rod **60** is positioned within the coupling element **14,** the top locking nut **16** is threaded onto the threading **62** of the upwardly extending members **54** and **56.** The nut **16** is then screwed down onto the coupling element **14** until the lower surface **66** of the engaging portion **65** of the nut **16** seats against the top surface of the rod **60**. As the nut **16** descends onto the coupling element **14,** the rod **60** is driven downward by the engaging portion **65** of the nut **16,** causing the rod **60** to engage the head **18** of the screw **12** and to push the head portion **18** of the screw **18** downward pressingly engaging it within the socket **40** of the coupling element **14.** This downward translation permits the bottom of the rod **60** to seat against the bottom surface **50** of the channels **48,** and causes the head **18** of the screw **12** to be crush locked to the inwardly curved surface **40** of the coupling element **14.** The force also engages the teeth **72** of the nut **16** into the rod **60** providing additional frictional engagement between the coupling element 14 and the rod **60.** As such, the downward force of the bottom surface **66** of the nut **16** against the rod **60,** as well as the teeth **72** and the counter-force provided by the bottom surface **50** of the channels **48** causes the rod **60** to be locked. This locking prevents the rod **60** from sliding relative to the assembled vertebral alignment assembly **10,** locking the rod **60** to the coupling element **14,** as well as the screw **12** to the coupling element **14.**

In addition to these basic components, fixation hardware could also be provided to fix the spine into the desired alignment. The fixation hardware may comprise clamps, which are designed to mate with the top or side of the pedicle screw, bendable fixation rods or plates, which run between the clamps on the various pedicle screws attached either to different vertebral bodies or at different points on a single vertebral body, and bolts, also designed to mate with the clamps such that the clamps can be tightened onto and fix the fixation rods into place. In addition, as shown in FIG. 6 the fixation hardware may comprise crosslinks of any design suitable for attachment to the alignment assembly **10** of the current invention. For example, although the crosslink shown in FIG. 6 is of fixed dimension, slotted crosslinks may also be used for applications in which the distance between the fixation points of the crosslink must be changed. In such an embodiment, the openings in the crosslink for attaching it to the alignment assembly **10** (which are shown as simple holes in FIG. 6) comprise elongated slots such that the crosslink may be slid relative to the alignment assembly **10** along the length of the slot, thereby allowing for some degree of adjustment in the position of the crosslink relative to the alignment assembly.

All of the above components, including the fixation hardware can be made of any suitable surgical material, such as, for example, stainless steel or titanium.

## Claims

1. A vertebral alignment/fixation assembly (10) comprising:
a screw (12) comprising an elongated threaded shaft (22) defining a screw axis with a substantially hemispherical head (18) having a driver engaging recess (32) disposed therein arranged at one end of the shaft (22) and a tapered tip (26) arranged at the other end of the shaft (22);
a coupling element (14) comprising a cylindrical body having an exterior threading (62) disposed on said upper surface (38) and defining an axial bore (34) through which said screw (12) may be inserted, said bore (34) having a narrowing inwardly curve lower surface (40) defining a socket designed to engage the substantially hemispherical head (18) of the screw (12) such that the head (18) of the screw (12) is prevented from passing therethrough and such that the screw (12) may be rotated therein to adjust the angle between the axis of the screw (12) and the axis of the bore (34), said coupling element (14) further comprising a pair of opposing upper channels (48) formed in the top of said coupling element (14) such that an elongated rod (60) may be received therein; and
a securing nut (16), mateable with said external threading (62), the nut (16) having an engaging portion (65) designed to fit within the bore (34) to engage the elongated rod (60),
**characterised in that** the coupling element (14) further comprises a pair of opposing lower channels (63) formed in the bottom of said coupling element (14) designed to allow the shaft (22) of the screw (12) to move therethrough, the lower channels (63) being designed such that the angle between the axis of the screw (12) and the axis of the coupling element (14) can be adjusted to at least 90 degrees, and **in that** the engaging portion (65) of the securing nut (16) further comprises a plurality of engaging teeth (72) designed to frictionally lock the rod (60) within the upper channel (48) of the coupling element (14).

2. A vertebral alignment/fixation assembly (10) according to claim 1, wherein the angles between the axis of the screw (12) and the axis of the coupling element (14) can be adjusted to at least 100 degrees.

3. A vertebral alignment/fixation assembly (10) according to claim 1 or claim 2, wherein the assembly is made of stainless steel.

4. A vertebral alignment/fixation assembly (10) according to any one of claims 1-3, wherein the recess (32) is designed to receive a screw driver from the group consisting of: flat-headed, phillips head, allen wrench, and square headed.

5. A vertebral alignment/fixation assembly (10) according to any preceding claim, wherein the nut (16) further comprises an axial recess (70) identical to the recess (32) disposed on the screw head (18).

6. A vertebral alignment/fixation assembly (10) according to claim 5, wherein the nut recess (70) is designed to receive a screw driver from the group consisting of: flat-headed, phillips head, allen wrench, and square headed.

7. A vertebral alignment/fixation assembly (10) according to any preceding claim, wherein the screw (12) further comprises a slotted groove (30) disposed longitudinally along the tip (26) and designed to allow the screw (12) to be self-tapped.

8. A vertebral alignment/fixation system comprising:
at least one elongated rod (60);
and at least one vertebral alignment/fixation assembly (10) according to any preceding claim.

9. A vertebral alignment/fixation system according to claim 8, wherein the system components are made of stainless steel.

10. A vertebral alignment/fixation system according to claim 8 or claim 9, further comprising at least one piece of fixation hardware designed to engage the at least one vertebral alignment/fixation assembly, the fixation hardware selected from the group consisting of: crosslinks, clamps, plates and rods.

## Patentansprüche

1. Wirbelausrichtungs-/ Fixierungsanordnung (10), umfassend:
eine Schraube (12) umfassend einen länglichen Gewindeschaft (22) definierend eine Schraubenachse mit einem im Wesentlichen halbkugelförmigen Kopf (18) mit einer darin angeordneten Drehereingriffsausnehmung (32) angeordnet an einem Ende des Schaftes (22) und eine verjüngte Spitze (26) angeordnet am anderen Ende des Schaftes (22);
ein Koppelelement (14) umfassend einen zylindrischen Körper mit einem Außengewinde (62) angeordnet an der oberen Oberfläche (38) und eine axiale Bohrung (34) definierend, durch die die Schraube (12) eingeführt werden kann, wobei die Bohrung (34) eine sich verengende, nach innen gekrümmte untere Oberfläche (40) aufweist, eine Buchse definierend, ausgelegt zur Ineingriffnahme des im Wesentlichen halbkugelförmigen Kopfes (18) der Schraube (12), so dass der Kopf (18) der Schraube (12) daran gehindert wird, dort hindurch zu gehen, und so dass die Schraube (12) darin gedreht werden kann, um den Winkel zwischen der Achse der Schraube (12) und der Achse der Bohrung (34) zu verstellen, wobei das Koppelelement (14) weiterhin ein Paar gegenüberliegender oberer Kanäle (48) umfaßt, die so in der Oberseite des Koppelelements (14) ausgebildet sind, dass ein länglicher Stab (60) darin aufgenommen werden kann; und
eine Sicherungsmutter (16), die mit dem Außengewinde (62) in Eingriff kommen kann, wobei die Mutter (16) einen Eingriffsabschnitt (65) aufweist, der so ausgelegt ist, dass er in die Bohrung (34) passt, um den länglichen Stab in (60) Eingriff zu nehmen,
**dadurch gekennzeichnet, dass** das Koppelelement (14) weiterhin ein Paar gegenüberliegender unterer Kanäle (63) umfaßt, die im Boden des Koppelelements (14) ausgebildet und so ausgelegt sind, dass sich der Schaft (22) der Schraube (12) dort hindurch bewegen kann, wobei die unteren Kanäle (63) derart ausgelegt sind, dass der Winkel zwischen der Achse der Schraube (12) und der Achse des Koppelelements (14) auf mindestens 90 Grad verstellt werden kann und dass der Eingriffsabschnitt (65) der Sicherungsmutter (16) weiterhin mehrere Eingriffszähne (72) umfaßt, die so ausgelegt sind, dass sie den Stab (60) reibungsmäßig in dem oberen Kanal (48) des Koppelelements (14) verriegeln.

2. Wirbelausrichtungs-/ Fixierungsanordnung (10) nach Anspruch 1, wobei die Winkel zwischen der Achse der Schraube (12) und der Achse des Koppelelements (14) auf mindestens 100 Grad verstellt werden können.

3. Wirbelausrichtungs-/ Fixierungsanordnung (10) nach Anspruch 1 oder 2, wobei die Baugruppe aus rostfreiem Stahl hergestellt ist.

4. Wirbelausrichtungs-/ Fixierungsanordnung (10) nach einem der Ansprüche 1-3, wobei die Ausnehmung (32) ausgelegt ist zum Aufnehmen eines Schraubendrehers aus der Gruppe bestehend aus: Flachkopf-, Phillipskopf-, Inbus- und Vierkantkopfschraubendreher.

5. Wirbelausrichtungs-/ Fixierungsbauanordnung (10) nach einem vorhergehenden Anspruch, wobei die Mutter (16) weiterhin eine axiale Ausnehmung (70) identisch mit der an dem Schraubenkopf (18) angeordneten Ausnehmung (32) umfaßt.

6. Wirbelausrichtungs-/ Fixierungsbauanordnung (10) nach Anspruch 5, wobei die Schraubenausnehmung (70) ausgelegt ist zum Aufnehmen eines Schraubendrehers aus der Gruppe bestehend aus: Flachkopf-, Phillipskopf-, Inbus- und Vierkantkopfschraubendreher.

7. Wirbelausrichtungs-/ Fixierungsanordnung (10) nach einem vorhergehenden Anspruch, wobei die Schraube (12) weiterhin eine Schlitznut (30) umfaßt, die in Längsrichtung entlang der Spitze (26) angeordnet und ausgelegt ist, dass die Schraube (12) selbstschneidend ist.

8. Wirbelausrichtungs-/ Fixierungssystem, umfassend:
mindestens einen länglichen Stab (60);
und mindestens eine Wirbelausrichtungs-/ Fixierungsanordnung nach einem vorhergehenden Anspruch.

9. Wirbelausrichtungs-/ Fixierungssystem nach Anspruch 8, wobei die Systemkomponenten aus rostfreiem Stahl hergestellt sind.

10. Wirbelausrichtungs-/ Fixierungssystem nach Anspruch 8 oder 9, weiterhin umfassend mindestens ein Teil Fixierungshardware, das ausgelegt ist zur Ineingriffnahme der mindestens einen Wirbelausrichtungs-/ Fixierungsanordnung, wobei die Fixierungshardware ausgewählt ist aus der Gruppe bestehend aus: Querverbindungen, Klammern, Platten und Stäben.

## Revendications

1. Ensemble d'alignement/fixation vertébral (10) comprenant :
une vis (12) comprenant une tige filetée allongée (22) définissant un axe de vis ayant une tête sensiblement hémisphérique (18) comportant un renfoncement d'engrènement de tournevis (32) agencé à une extrémité de la tige (22) et une pointe conique (26) agencée à l'autre extrémité de la tige (22) ;
un élément d'accouplement (14) comprenant un corps cylindrique ayant un filetage extérieur (62) disposé sur ladite surface supérieure (38) et définissant un alésage axial (34) à travers lequel ladite vis (12) peut être insérée, ledit alésage (34) ayant une surface inférieure courbe s'amenuisant vers l'intérieur (40) définissant une douille conçue pour s'engrener avec la tête sensiblement hémisphérique (18) de la vis (12) de façon à empêcher la tête (18) de la vis (12) de la traverser et de telle sorte que la vis (12) puisse être tournée à l'intérieur pour régler l'angle entre l'axe de la vis (12) et l'axe de l'alésage (34), ledit élément d'accouplement (14) comprenant en outre une paire de rainures supérieures opposées (48) formées dans la partie haute dudit élément d'accouplement (14) de telle sorte qu'une tige allongée (60) puisse être reçue à l'intérieur ; et
un écrou de fixation (16), pouvant être accouplé avec ledit filetage externe (62), l'écrou (16) ayant une partie d'engrènement (65) conçue pour s'engager dans l'alésage (34) afin de s'engrener avec la tige allongée (60),
**caractérisé en ce que** l'élément d'accouplement (14) comprend en outre une paire de rainures inférieures opposées (63) formées dans la partie basse dudit élément d'accouplement (14) conçues pour permettre à la tige (22) de la vis (12) de se mouvoir à travers lui, les rainures inférieures (63) étant conçues de telle sorte que l'angle entre l'axe de la vis (12) et l'axe de l'élément d'accouplement (14) puisse être réglé à au moins 90 degrés, et **en ce que** la partie d'engrènement (65) de l'écrou de fixation (16) comprend en outre une pluralité de dents d'engrènement (72) conçues pour verrouiller par friction la tige (60) dans la rainure supérieure (48) de l'élément d'accouplement (14).

2. Ensemble d'alignement/fixation vertébral (10) selon la revendication 1, dans lequel les angles entre l'axe de la vis (12) et l'axe de l'élément d'accouplement (14) peuvent être réglés à au moins 100 degrés.

3. Ensemble d'alignement/fixation vertébral (10) selon la revendication 1 ou 2, l'ensemble étant réalisé en acier inoxydable.

4. Ensemble d'alignement/fixation vertébral (10) selon l'une quelconque des revendications 1 à 3, dans lequel le renfoncement (32) est conçu pour recevoir un tournevis compris dans le groupe consistant en tournevis pour vis à tête fraisée, tournevis pour vis cruciforme, clé hexagonale et tournevis pour vis à tête carrée.

5. Ensemble d'alignement/fixation vertébral (10) selon l'une quelconque des revendications précédentes, dans lequel l'écrou (16) comprend en outre un renfoncement axial (70) identique au renfoncement (32) disposé sur la tête de vis (18).

6. Ensemble d'alignement/fixation vertébral (10) selon la revendication 5, dans lequel le renfoncement d'écrou (70) est conçu pour recevoir un tournevis compris dans le groupe consistant en tournevis pour vis à tête fraisée, tournevis pour vis cruciforme, clé hexagonale et tournevis pour vis à tête carrée.

7. Ensemble d'alignement/fixation vertébral (10) selon l'une quelconque des revendications précédentes, dans lequel la vis (12) comprend en outre une gorge fendue (30) disposée longitudinalement le long de la pointe (26) et conçue pour permettre un auto-taraudage de la vis (12).

8. Système d'alignement/fixation vertébral comprenant :
au moins une tige allongée (60) ;
et au moins un ensemble d'alignement/fixation vertébral (10) selon l'une quelconque des revendications précédentes.

9. Système d'alignement/fixation vertébral selon la revendication 8, dans lequel les composants du système sont réalisés en acier inoxydable.

10. Système d'alignement/fixation vertébral selon la revendication 8 ou 9, comprenant en outre au moins une pièce de matériel de fixation conçue pour s'engrener avec l'au moins un ensemble d'alignement/fixation vertébral, le matériel de fixation étant sélectionné dans le groupe consistant en traverses, brides, plaques et tiges.
